Europäisches Patentamt

(19) European Patent Office

Office européen des brevets

(11) EP 0 713 724 B1

(12) # EUROPEAN PATENT SPECIFICATION

(45) Date of publication and mention
of the grant of the patent:
**19.05.1999 Bulletin 1999/20**

(21) Application number: **94923092.4**

(22) Date of filing: **09.08.1994**

(51) Int. Cl.[6]: **B01J 23/887**, C07C 255/08,
C07C 253/24

(86) International application number:
**PCT/JP94/01312**

(87) International publication number:
**WO 95/04593 (16.02.1995 Gazette 1995/08)**

(54) **AMMOXIDATION CATALYST COMPOSITION AND PROCESS FOR PRODUCING
ACRYLONITRILE OR METHACRYLONITRILE BY USING THE SAME**

AMMOXYDATIONSKATALYSATORZUSAMMENSETZUNG UND VERFAHREN ZUR HERSTELLUNG
VON ACRYLONITRIL ODER METHACRYLONITRO UNTER DEREN ANWENDUNG

COMPOSITION D'UN CATALYSEUR D'AMMOXYDATION ET PROCEDE L'UTILISANT POUR
PRODUIRE DE L'ACRYLONITRILE OU DU METHACRYLONITRILE

(84) Designated Contracting States:
**DE ES GB IT NL**

(30) Priority: **10.08.1993 JP 198523/93**

(43) Date of publication of application:
**29.05.1996 Bulletin 1996/22**

(73) Proprietor:
**Asahi Kasei Kogyo Kabushiki Kaisha
Osaka-shi, Osaka 530-8205 (JP)**

(72) Inventors:
• **MIDORIKAWA, Hideo
Okayama 710 (JP)**

• **SOMEYA, Ken
Okayama 710 (JP)**

(74) Representative:
**Blake, John Henry Francis
Brookes & Martin
High Holborn House
52/54 High Holborn
London WC1V 6SE (GB)**

(56) References cited:
**JP-A-58 067 349        JP-A-58 143 843
LU-A-   49 464**

**Description**

Background of the Invention

Technical field

The present invention relates to an ammoxidation catalyst composition, and a process for producing acrylonitrile or methacrylonitrile using the same. More particularly, the present invention is concerned with an ammoxidation catalyst composition for use in the process for producing acrylonitrile or methacrylonitrile by reacting propylene with, or reacting isobutene or tert-butanol with a molecular oxygen-containing gas and ammonia, the ammoxidation catalyst composition comprising an oxide catalyst composition comprised of molybdenum, bismuth, cerium, iron, nickel, at least one element selected from magnesium and zinc, and at least one element selected from potassium, rubidium and cesium, wherein the atomic ratio of the sum of bismuth and cerium, relative to twelve atoms of molybdenum, is from 0.5 to 2 and the atomic ratio of cerium to the sum of bismuth and cerium is from 0.6 to 0.8. By use of such an ammoxidation catalyst composition, not only can acrylonitrile or methacrylonitrile be produced in high yield, but also a lowering of the yield of acrylonitrile or methacrylonitrile can be effectively suppressed even after the operation of the production process has been conducted for a prolonged period of time. The present invention is also concerned with a process for producing acrylonitrile or methacrylonitrile using such an ammoxidation catalyst composition.

Prior art

It has been well known to produce acrylonitrile or methacrylonitrile by ammoxidation of propylene, or of isobutene or tert-butanol, namely, a reaction of propylene with, or of isobutene or tert-butanol with a molecular oxygen-containing gas and ammonia. A number of proposals have been made with respect to catalysts for use in the ammoxidation of propylene, or of isobutene or tert-butanol. For example, U.S. Patent No. 3,226,422 proposes an oxide catalyst containing molybdenum, bismuth and iron, and Examined Japanese Patent Application Publication No. 38-19111 proposes an oxide catalyst containing antimony and iron. Further, various improvements have been proposed with respect to these ammoxidation catalyst systems.

For example, each of British Patent No. 1,445,512 and U.S. Patent No. 4,746,753 discloses a catalyst containing an alkali metal and thallium in addition to molybdenum, bismuth and cerium. Examined Japanese Patent Application Publication No. 61-43094 discloses a catalyst comprising molyodenum, tungsten, bismuth and cerium. U.S. Patent No. 3,969,390 discloses an oxide catalyst containing at least one element selected from iron, chromium, aluminum and bismuth, in addition to molyodenum, tellurium and cerium. U.S. Patent No. 4,192,776 discloses an oxide catalyst containing, in addition to molybdenum, bismuth and iron, at least one element selected from nickel and cobalt, and at least one element selected from an alkali metal, a rare earth element, tantalum and niobium. U.S. Patent No. 4,443,556 discloses an oxide catalyst containing molyodenum, bismuth and iron as essential elements, and also containing at least one element selected from cerium, lanthanum, neodymium, praseodymium, samarium, europium and gadolinium, and at least one element selected from potassium, rubidium and cesium. Unexamined Japanese Patent Application Laid-Open Specification No. 59-204163 discloses a catalyst containing, in addition to molybdenum, bismuth, phosphorus and silicon, at least two elements selected from iron, cobalt, nickel, copper, zirconium and potassium, and at least one element selected from manganese, cerium, thorium, yttrium, lanthanum and thallium.

Further, each of U.S. Patent No. 5,093,299, U.S. Patent No. 5,175,334 and U.S. Patent No. 5,212,137 discloses a catalyst containing molybdenum, bismuth, iron, nickel, magnesium, potassium and cesium as essential elements and optionally containing cobalt, manganese, chromium, phosphorus, antimony, tellurium, sodium, cerium and/or tungsten, and a process for producing acrylonitrile or methacrylonitrile using such a catalyst. However, it is noted that in the working examples of the above patent documents, a catalyst containing cerium is not used.

The catalysts disclosed in the above-mentioned patent documents are greatly improved in respect of the yield of acrylonitrile or methacrylonitrile at the initial stage of reaction. However, those catalysts are still unsatisfactory in respect of the yield of acrylonitrile or methacrylonitrile when the operation of the production process is conducted for a prolonged period of time.

Disclosure of the Invention

In the above situations, the present inventors have made extensive and intensive studies toward developing a catalyst for use in ammoxidation, which is free from the above-mentioned problem, and which can be advantageously used not only for producing acrylonitrile or methacrylonitrile in high yield, but also for performing a stable ammoxidation reaction even when the operation of the production process is conducted for a prolonged period of time, so that a lowering of the yield of acrylonitrile or methacrylonitrile is small. As a result, it has been found that when an oxide catalyst composition, which is represented by the formula (I):

$$Mo_{12}(Bi_{1-a}Ce_a)_bFe_cNi_dX_eY_fO_g \tag{I}$$

wherein:

X is at least one element selected from magnesium and zinc,
Y is at least one element selected from potassium, rubidium and cesium,
a is the atomic ratio of cerium to the sum of bismuth and cerium,
b is the atomic ratio of the sum of bismuth and cerium, relative to twelve atoms of molybdenum, and c, d, e, f and g are, respectively, the atomic ratios of iron, nickel, X, Y and oxygen, relative to twelve atoms of molybdenum, wherein

a is a number of from 0.6 to 0.8,
b is a number of from 0.5 to 2,
c is a number of from 0.1 to 3,
d is a number of from 4 to 10,
e is a number of from 0 to 3,
f is a number of from 0.01 to 2, and
g is a number determined by the valence requirements of the other elements present,

is used as an ammoxidation catalyst in the process for producing acrylonitrile or methacrylonitrile by reacting propylene with, or reacting isobutene or tert-butanol with a molecular oxygen-containing gas and ammonia, not only can acrylonitrile or methacrylonitrile be produced in high yield, but also the ammoxidation reaction can be stably conducted even when the operation of the production process is conducted for a prolonged period of time, so that a lowering of the yield of acrylonitrile or methacrylonitrile is very small. The present invention has been made, based on this novel finding.

Accordingly, it is an object of the present invention to provide an ammoxidation catalyst composition, by use of which not only can acrylonitrile or methacrylonitrile be produced in high yield, but also the ammoxidation reaction can be stably conducted even when the operation of the production process is conducted for a prolonged period of time, so that a lowering of the yield of acrylonitrile or methacrylonitrile is very small.

It is another object of the present invention to provide a process for producing acrylonitrile or methacrylonitrile using the above-mentioned novel ammoxidation catalyst composition.

The foregoing and other objects, features and advantages of the present invention will be apparent from the following detailed description and appended claims.

Detailed Description of the Invention

In one aspect of the present invention, there is provided an ammoxidation catalyst composition comprising an oxide catalyst composition represented by the formula (I):

$$Mo_{12}(Bi_{1-a}Ce_a)_bFe_cNi_dX_eY_fO_g \tag{I}$$

wherein:

X is at least one element selected from magnesium and zinc,
Y is at least one element selected from potassium, rubidium and cesium,
a is the atomic ratio of cerium to the sum of bismuth and cerium,
b is the atomic ratio of the sum of bismuth and cerium, relative to twelve atoms of molybdenum, and c, d, e, f and g are, respectively, the atomic ratios of iron, nickel, X, Y and oxygen, relative to twelve atoms of molybdenum, wherein

a is a number of from 0.6 to 0.8,
b is a number of from 0.5 to 2,
c is a number of from 0.1 to 3,
d is a number of from 4 to 10,
e is a number of from 0 to 3,
f is a number of from 0.01 to 2, and
g is a number determined by the valence requirements of the other elements present.

The ammoxidation catalyst composition of the present invention has a characteristic feature in that the atomic ratio b of the sum of bismuth and cerium, relative to twelve atoms of molybdenum, is from 0.5 to 2, preferably 0.7 to 1.8, and the atomic ratio a of cerium to the sum of bismuth and cerium is from 0.6 to 0.8. When b is less than 0.5 or is more than 2, not only does the yield of acrylonitrile or methacrylonitrile at the initial stage of reaction become low, but also the ammoxidation reaction becomes unstable. When a is less than 0.6, although the yield of acrylonitrile or methacrylonitrile is good at the initial stage of reaction, not only is the ammoxidation reaction unstable but the yield of acrylonitrile or methacrylonitrile is also drastically lowered with the lapse of operation time. On the other hand, when a is more than 0.8, the yield of acrylonitrile or methacrylonitrile is disadvantageously lowered even at the initial stage of reaction. With respect to c, d, e and f, which are, respectively, the atomic ratios of iron, nickel, X (which is at least one element selected from magnesium and zinc) and Y (which is at least one element selected from potassium, rubidium and cesium), relative to twelve atoms of molybdenum, c is from 0.1 to 3, preferably from 0.5 to 2.5; d is from 4 to 10, preferably from 5 to 8; e is from 0 to 3, preferably from 0.1 to 2.5; and f is from 0.01 to 2, preferably from 0.02 to 1.

With respect to a carrier which can be used to support thereon the oxide catalyst composition of the present invention, oxides, such as silica, alumina, titania and zirconia, can be employed. Of these oxides, silica is preferred. Silica is inherently inert, differing from other carrier materials, and can serve as an excellent binder for the ingredients of the oxide catalyst composition without impairing the selectivity of the oxide catalyst composition and serve to impart the resulting catalyst composition with a high attrition resistance. The amount of carrier to be used may be in the range of 30 to 70 % by weight, preferably 40 to 60 % by weight, based on the total weight of the oxide catalyst composition and the carrier.

The ammoxidation catalyst composition of the present invention can be produced by a conventional method. For example, the ammoxidation catalyst composition can be produced by a method comprising the steps of (1) preparing a slurry of starting materials, (2) spray-drying the slurry prepared in step (1) above to obtain a dried particulate catalyst precursor, and (3) subjecting the dried particulate catalyst precursor obtained in step (2) above to calcination and firing, successively.

Hereinbelow, explanation is made with respect to a preferred embodiment of the above-mentioned method for producing the ammoxidation catalyst composition of the present invention, which comprises steps (1), (2) and (3), above.

In step (1), a slurry of starting materials is prepared. In the starting materials, each of the elements (which are to be incorporated into a catalyst composition), i.e., molybdenum, bismuth, cerium, iron, nickel, magnesium, zinc, potassium, rubidium and cesium, may be present in the form of an ammonium salt, a nitrate, a chloride, a sulfate and/or an organic acid salt, which are soluble in water or nitric acid. Especially, it is preferred that a molybdenum source be in the form of an ammonium salt, and that each of bismuth, cerium, iron, nickel, magnesium, zinc, potassium, rubidium and cesium be used in the form of a nitrate.

As mentioned above, in the ammoxidation catalyst composition of the present invention, an oxide, such as silica, alumina, titania or zirconia, can be employed as a carrier for the oxide catalyst composition represented by formula (I). Of the above oxides, silica is most advantageously used. As the source of silica, a silica sol is preferred.

The slurry of starting materials can be prepared by, for example, adding a solution of nitrates of component metals except molybdenum (i.e., sources of bismuth, cerium, iron, nickel, magnesium, zinc, potassium, rubidium and cesium) in water or in aqueous nitric acid to a silica sol, followed by addition of an aqueous ammonium molybdate solution. Alternatively, for preparing the slurry of starting materials, the above-mentioned aqueous ammonium molybdate solution may be first added to a silica sol, followed by addition of the above-mentioned solution of nitrates of component metals except molybdenum.

In step (2), the slurry obtained in step (1) above is subjected to spray drying, to thereby obtain a quasispherical particulate catalyst precursor. The spray drying of the slurry can be generally conducted by centrifugation, two-phase flow nozzle method or high pressure nozzle method to obtain a dried particulate catalyst precursor.

In this instance, it is preferred to use air which has been heated by an electric heater, steam or the like, as a heat source for drying. In this case, it is preferred that the temperature at an entrance to the dryer of the spray dryer be from 100 to 400 °C, preferably 150 to 300 °C.

In step (3), the dried particulate catalyst precursor obtained in step (2) above is calcined and finally fired to thereby obtain a desired oxide catalyst composition. The dried particulate catalyst is calcined at a temperature of from 150 to 500 °C, and then subjected to firing at a temperature of from 500 to 750 °C, preferably 550 to 700 °C for 1 to 20 hours. For the calcination and firing, a kiln, such as a rotary kiln, a tunnel kiln or a muffle kiln, can be used.

When the ammoxidation catalyst composition of the present invention comprises an oxide catalyst composition supported on a carrier (preferably silica), it is preferred that the particle diameter distribution of the ammoxidation catalyst composition be within the range of from 10 to 150 μm.

The process for producing acrylonitrile or methacrylonitrile by reacting propylene with, or reacting isobutene or tert-butanol with a molecular oxygen-containing gas and ammonia in the presence of an ammoxidation catalyst composition of the present invention may be conducted either in a fluidized bed reactor or in a fixed bed reactor. However, a fluidized bed reactor is preferred.

Propylene, or isobutene or tert-butanol and ammonia to be used in the process of the present invention need not necessarily be of so high purity but may be of a commercial grade. As a source of oxygen, air is usually employed. Gas having an increased oxygen content, such as a gaseous mixture of air and oxygen, is also usable.

In the process of the present invention, it is advantageous that the molar ratios of propylene, or isobutene or tert-butanol: ammonia: air be in the range of 1 : 0.8 to 1.4 : 7 to 12, preferably 1 : 0.9 to 1.3 : 8 to 11. The reaction temperature may be 350 to 550 °C, preferably 400 to 500 °C. The reaction may usually be conducted under a pressure of from atmospheric pressure to a pressure of 3 atm. The time of contact between gaseous mixture of the raw materials and the catalyst composition (contact time) may be from 0.5 to 20 sec • g/cc, preferably from 1 to 10 sec • g/cc.

Thus, in another aspect of the present invention, there is provided a process for producing acrylonitrile from propylene, or methacrylonitrile from isobutene or tert-butanol, by ammoxidation of the propylene or of the isobutene or tert-butanol, which comprises reacting propylene with, or reacting isobutene or tert-butanol with a molecular oxygen-containing gas and ammonia at a temperature of from 350 °C to 550 °C under a pressure of from atmospheric pressure to 3 atm in the presence of an ammoxidation catalyst composition comprising an oxide catalyst composition represented by the formula (I):

$$Mo_{12}(Bi_{1-a}Ce_a)_bFe_cNi_dX_eY_fO_g \qquad\qquad (I)$$

wherein:

X is at least one element selected from magnesium and zinc,
Y is at least one element selected from potassium, rubidium and cesium,
a is the atomic ratio of cerium to the sum of bismuth and cerium,
b is the atomic ratio of the sum of bismuth and cerium, relative to twelve atoms of molybdenum, and c, d, e, f and g are, respectively, the atomic ratios of iron, nickel, X, Y and oxygen, relative to twelve atoms of molybdenum, wherein

a is a number of from 0.6 to 0.8,
b is a number of from 0.5 to 2,
c is a number of from 0.1 to 3,
d is a number of from 4 to 10,
e is a number of from 0 to 3,
f is a number of from 0.01 to 2, and
g is determined by the valence requirements of the other elements present.

As mentioned above, by use of the ammoxidation catalyst composition of the present invention in the process for producing acrylonitrile or methacrylonitrile by reacting propylene with, or reacting isobutene or tert-butanol with a molecular oxygen-containing gas and ammonia, not only can acrylonitrile or methacrylonitrile be produced in high yield, but also the ammoxidation reaction can be stably conducted for a prolonged period of time, so that even after the operation of the production process has been conducted for a period of time as long as about one month, a lowering of the yield of acrylonitrile or methacrylonitrile is as small as 0.5 % or less, relative to the yield of acrylonitrile or methacrylonitrile at the initial stage of reaction.

BEST MODE FOR CARRYING OUT THE INVENTION

Hereinbelow, the present invention will be described in more detail with reference to the following Examples and Comparative Examples, but they should not be construed as limiting the scope of the present invention.

In the following Examples and Comparative Examples, the conversion and yield used for evaluating the results of the reaction are defined as follows:

$$\text{Conversion (\%)} = \frac{\text{mole of propylene, isobutene or tert-butanol reacted}}{\text{mole of propylene, isobutene or tert-butanol fed}} \times 100$$

$$\text{Yield (\%)} = \frac{\text{mole of acrylonitrile or methacrylonitrile formed}}{\text{mole of propylene, isobutene or tert-butanol fed}} \times 100$$

A stainless steel (SUS 304) fluidized bed reactor having an outer diameter of 3 inches was used as a reaction apparatus. The reaction pressure (P) was maintained at 0.5 kg/cm$^2$ • G, and the reaction temperature (T) was maintained at 430 °C. The amount (W) of a catalyst charged in the reactor was 1,000 to 2,000 g, and the total flow rate (F) of raw material gases introduced into the reactor was 100 to 150 cc/sec in terms of the volume per unit time under normal temperature and pressure (N.T.P) conditions.

The contact time is defined by the following formula:

$$\text{Contact time (sec} \cdot \text{g/cc)} = (W/F) \times 273/(273+T) \times (1.03+P)/1.03.$$

The compositions of raw material gases introduced into the reactor were as follows:

for an ammoxidation reaction of propylene:

propylene/ammonia/air = 1/1.1/8.0-10.0.

for an ammoxidation reaction of isobutene or tert-butanol:

isobutene or tert-butanol/ammonia/air = 1/1.2/9.0-10.5.

Example 1

An ammoxidation catalyst composition, composed of oxides supported on 50 % by weight, based on the total weight of the oxides and silica, of silica, having a structure represented by the formula:

$$Mo_{12}Bi_{0.20}Ce_{0.40}Fe_{2.0}Ni_{5.6}Mg_{2.2}K_{0.07}Cs_{0.04},$$

was prepared as follows.

38.6 g of bismuth nitrate [$Bi(NO_3)_3 \cdot 5H_2O$], 69.0 g of cerium nitrate [$Ce(NO_3)_3 \cdot 6H_2O$], 321.2 g of iron nitrate [$Fe(NO_3)_3 \cdot 9H_2O$], 647.6 g of nickel nitrate [$Ni(NO_3)_2 \cdot 6H_2O$], 224.2 g of magnesium nitrate [$Mg(NO_3)_2 \cdot 6H_2O$], 2.82 g of potassium nitrate [$KNO_3$] and 3.10 g of cesium nitrate [$CsNO_3$] were dissolved in 755.4 g of a 17.9 wt% aqueous nitric acid solution. The resultant solution was added to 3,333.4 g of a silica sol having a $SiO_2$ content of 30 wt%, to obtain a mixture. To the thus obtained mixture was added a solution of 842.4 g of ammonium paramolybdate [$(NH_4)_6Mo_7O_{24} \cdot 4H_2O$] in 1,696.6 g of water, to thereby obtain a slurry. The slurry thus obtained was fed to a parallel flow type spray-drying apparatus, in which the slurry was atomized by means of a sprayer having a dish type rotor disposed above the central portion of a dryer of the spray-drying apparatus, and dried at about 200 °C, to thereby obtain a dried particulate catalyst precursor. The obtained dried particulate catalyst precursor was calcined in an electric kiln at 400 °C for 1 hour and then subjected to firing at 590 °C for 2 hours, thereby obtaining a catalyst composition supported on the silica.

Using 1,400 g of the obtained catalyst composition supported on the silica, an ammoxidation reaction of propylene was conducted. The contact time in the ammoxidation was 6.7 sec • g/cc. Results of the reaction were evaluated at time points of 100 hours, 700 hours and 1,400 hours after the start of reaction. As a result, it was found that, 100 hours after the start of reaction, the conversion of propylene was 99.8 %, and the yield of acrylonitrile was 80.8 %; that, 700 hours after the start of reaction, the conversion of propylene was 99.7 %, and the yield of acrylonitrile was 80.5 %; and that, 1,400 hours after the start of reaction, the conversion of propylene was 99.6 %, and the yield of acrylonitrile was 80.2 %.

Examples 2 to 9 and Comparative Examples 1 to 4

Ammoxidation catalyst compositions, composed of oxides supported on 50 % by weight, based on the total weight of the oxides and silica, of silica, having the respective compositions indicated in Table 1 were prepared in substantially the same manner as in Example 1. Using the obtained catalyst compositions individually, ammoxidation reactions of propylene were conducted. Results are shown in Table 1.

## Table 1 (to be continued)

| | Mo | Bi | Ce | Fe | Ni | X | Y | a | b |
|---|---|---|---|---|---|---|---|---|---|
| Example 1 | 12 | 0.20 | 0.40 | 2.0 | 5.6 | Mg 2.2 | K 0.07  Cs 0.04 | 0.67 | 0.60 |
| Example 2 | 12 | 0.45 | 0.90 | 1.8 | 5.0 | Mg 2.0 | K 0.09  Rb 0.05 | 0.67 | 1.35 |
| Example 3 | 12 | 0.45 | 0.90 | 1.8 | 5.0 | Zn 2.0 | K 0.09  Rb 0.05 | 0.67 | 1.35 |
| Example 4 | 12 | 0.60 | 1.20 | 1.6 | 4.8 | Mg 1.9 | K 0.11  Rb 0.05 | 0.67 | 1.80 |
| Example 5 | 12 | 0.27 | 1.08 | 1.8 | 5.0 | Mg 2.0 | K 0.11  Cs 0.03 | 0.80 | 1.35 |
| Example 6 | 12 | 0.54 | 0.81 | 1.8 | 7.0 | 0 | K 0.10  Rb 0.04 | 0.60 | 1.35 |
| Example 7 | 12 | 0.34 | 1.01 | 2.2 | 4.1 | Mg 2.5 | K 0.20 | 0.75 | 1.35 |
| Example 8 | 12 | 0.45 | 0.90 | 1.8 | 5.0 | Mg 2.0 | Rb 0.15 | 0.67 | 1.35 |
| Example 9 | 12 | 0.39 | 0.96 | 2.0 | 5.3 | Mg 1.5 | Cs 0.13 | 0.71 | 1.35 |
| Comp. Ex. 1 | 12 | 0.83 | 1.67 | 1.5 | 4.1 | Mg 1.6 | K 0.09  Rb 0.05 | 0.67 | 2.50 |
| Comp. Ex. 2 | 12 | 1.35 | 0 | 2.0 | 5.5 | Mg 2.2 | K 0.09  Rb 0.05 | 0 | 1.35 |
| Comp. Ex. 3 | 12 | 0.68 | 0.68 | 1.8 | 5.0 | Mg 2.0 | K 0.09  Rb 0.05 | 0.50 | 1.36 |
| Comp. Ex. 4 | 12 | 0.14 | 1.21 | 1.8 | 5.0 | Mg 2.0 | K 0.09  Rb 0.05 | 0.90 | 1.35 |

Table 1 (continued)

| | Temperature (°C) for firing of catalyst composition | Contact time (sec·g/cc) | Results obtained 100 hrs after the start of reaction | | Results obtained 700 hrs after the start of reaction | | Results obtained 1400 hrs after the start of reaction | |
|---|---|---|---|---|---|---|---|---|
| | | | Conversion (%) | Yield (%) | Conversion (%) | Yield (%) | Conversion (%) | Yield (%) |
| Example 1 | 590 | 6.0 | 99.8 | 80.8 | 99.7 | 80.5 | 99.6 | 80.2 |
| Example 2 | 610 | 6.7 | 99.8 | 82.0 | 99.7 | 81.8 | 99.7 | 81.8 |
| Example 3 | 610 | 6.7 | 99.7 | 81.9 | 99.7 | 81.7 | 99.6 | 81.6 |
| Example 4 | 590 | 6.2 | 99.8 | 81.6 | 99.7 | 81.4 | 99.5 | 81.3 |
| Example 5 | 600 | 6.4 | 99.7 | 81.4 | 99.6 | 81.2 | 99.5 | 80.9 |
| Example 6 | 610 | 6.6 | 99.6 | 81.8 | 99.5 | 81.6 | 99.3 | 81.3 |
| Example 7 | 600 | 6.7 | 99.8 | 81.5 | 99.7 | 81.4 | 99.6 | 81.2 |
| Example 8 | 570 | 6.7 | 99.7 | 81.9 | 99.6 | 81.8 | 99.5 | 81.7 |
| Example 9 | 550 | 6.7 | 99.8 | 81.4 | 99.7 | 81.3 | 99.5 | 81.1 |
| Comp. Ex. 1 | 610 | 6.7 | 99.8 | 79.8 | 99.7 | 79.0 | - | - |
| Comp. Ex. 2 | 580 | 5.9 | 99.9 | 82.8 | 99.7 | 81.2 | 99.5 | 78.6 |
| Comp. Ex. 3 | 610 | 6.7 | 99.8 | 82.3 | 99.7 | 81.3 | 99.6 | 79.9 |
| Comp. Ex. 4 | 610 | 6.7 | 99.7 | 76.3 | 99.5 | 75.8 | - | - |

Note: "-" means that data were not available because the operation of the production process was discontinued 700 hours after the start of reaction.

Example 10

An ammoxidation catalyst composition, composed of oxides supported on 50 % by weight, based on the total weight of the oxides and silica, of silica, having a structure represented by the formula:

$$Mo_{12}Bi_{0.45}Ce_{0.90}Fe_{2.0}Ni_{5.0}Mg_{2.0}K_{0.45},$$

was prepared in substantially the same manner as in Example 1, except that the firing was conducted at 610 °C. Using 1,000 g of an obtained catalyst composition supported on silica, an ammoxidation reaction of tert-butanol was conducted at 430 °C. The contact time was 4.8 sec·g/cc. Results of the reaction were evaluated at time points of 100

8

hours, 700 hours and 1,400 hours after the start of reaction. As a result, it was found that, 100 hours after the start of reaction, the conversion of tert-butanol was 99.7 %, and the yield of methacrylonitrile was 71.9 %; that, 700 hours after the start of reaction, the conversion of tert-butanol was 99.6 %, and the yield of methacrylonitrile was 71.7 %; and that, 1,400 hours after the start of reaction, the conversion of tert-butanol was 99.4 %; and the yield of methacrylonitrile was 71.5 %.

Industrial Applicability

By use of the ammoxidation catalyst composition of the present invention in producing acrylonitrile from propylene, or methacrylonitrile from isobutene or tert-butanol, by ammoxidation of the propylene or of the isobutene or tert-butanol, not only can acrylonitrile or methacrylonitrile be produced in high yield, but also the ammoxidation reaction can be stably conducted even when the operation of the production process is conducted for a prolonged period of time, so that a lowering of the yield of acrylonitrile or methacrylonitrile, relative to the yield thereof at the initial stage of reaction, is very small.

**Claims**

1. An ammoxidation catalyst composition for use in producing acrylonitrile from propylene, or methacrylonitrile from isobutene or tert-butanol, by ammoxidation of said propylene or of said isobutene or tert-butanol, comprising an oxide catalyst composition represented by the formula (I):

$$Mo_{12}(Bi_{1-a}Ce_a)_bFe_cNi_dX_eY_fO_g \qquad (I)$$

wherein:

X is at least one element selected from magnesium and zinc,
Y is at least one element selected from potassium, rubidium and cesium,
a is the atomic ratio of cerium to the sum of bismuth and cerium,
b is the atomic ratio of the sum of bismuth and cerium, relative to twelve atoms of molybdenum, and c, d, e, f and g are, respectively, the atomic ratios of iron, nickel, X, Y and oxygen, relative to twelve atoms of molybdenum,
wherein

a is a number of from 0.6 to 0.8,
b is a number of from 0.5 to 2,
c is a number of from 0.1 to 3,
d is a number of from 4 to 10,
e is a number of from 0 to 3,
f is a number of from 0.01 to 2, and
g is a number determined by the valence requirements of the other elements present.

2. The catalyst composition according to claim 1, wherein b, c, d and f in formula (I) are, respectively, from 0.7 to 1.8, from 0.5 to 2.5, from 5 to 8, and from 0.02 to 1, relative to twelve atoms of molybdenum.

3. The catalyst composition according to claim 1 or 2, wherein e in formula (I) is from 0.1 to 2.5, relative to twelve atoms of molybdenum.

4. The catalyst composition according to any one of claims 1 to 3, wherein Y in formula (I) is potassium.

5. The catalyst composition according to any one of claims 1 to 3, wherein Y in formula (I) is rubidium.

6. The catalyst composition according to any one of claims 1 to 3, wherein Y in formula (I) is cesium.

7. The catalyst composition according to any one of claims 1 to 3, wherein Y in formula (I) is a mixture of at least two elements selected from the group consisting of potassium, rubidium and cesium.

8. The catalyst composition according to any one of claims 1 to 7, which further comprises silica as a carrier having said oxide catalyst composition supported thereon, wherein said silica carrier is present in an amount of from 30 to 70 % by weight, based on the total weight of said oxide catalyst composition and said silica carrier.

9. A process for producing acrylonitrile from propylene, or methacrylonitrile from isobutene or tert-butanol, by ammoxidation of said propylene or of said isobutene or tert-butanol, which comprises reacting propylene with, or reacting isobutene or tert-butanol with a molecular oxygen-containing gas and ammonia at a temperature of from 350 °C to 550 °C under a pressure of from atmospheric pressure to 3 atm in the presence of an ammoxidation catalyst composition as claimed in any one of claims 1 to 8.

10. The process according to claim 9, wherein said molecular oxygen-containing gas is air.

11. The process according to claim 9 or 10, wherein the molar ratios of propylene, or isobutene or tert-butanol: ammonia: oxygen are in the range of 1 : 0.8 to 1.4 : 1.4 to 2.4.

12. The process according to claim 9, 10 or 11, wherein said reaction is conducted in a fluidized bed reactor.

**Patentansprüche**

1. Ammoxidationskatalysatorzusammensetzung für die Verwendung bei der Herstellung von Acrylnitril aus Propylen oder von Methacrylnitril aus Isobuten oder tert-Butanol durch Ammoxidation des Propylens oder des Isobutens oder tert-Butanols, welche eine Oxidkatalysatorzusammensetzung enthält, die durch die Formel (I) dargestellt wird:

$$Mo_{12}(Bi_{1-a}Ce_a)_bFe_cNi_dX_eY_fO_g \qquad\qquad (I)$$

   worin:

   X mindestens ein unter Magnesium und Zink ausgewähltes Element ist,
   Y mindestens ein unter Kalium, Rubidium und Cäsium ausgewähltes Element ist,
   a das Atomverhältnis von Cer zu der Summe von Bismut und Cer ist,
   b das Atomverhältnis der Summe von Bismut und Cer, bezogen auf 12 Atome Molybdän, ist, und c, d, e, f und g die Atomverhältnisse von Eisen, Nickel, X, Y bzw. Sauerstoff, bezogen auf 12 Atome Molybdän, sind, wobei

   a eine Zahl von 0,6 bis 0,8 ist,
   b eine Zahl von 0,5 bis 2 ist,
   c eine Zahl von 0,1 bis 3 ist,
   d eine Zahl von 4 bis 10 ist,
   e eine Zahl von 0 bis 3 ist,
   f eine Zahl von 0,01 bis 2 ist und
   g eine Zahl ist, die durch die Wertigkeitserfordernisse der anderen vorhandenen Elemente bestimmt wird.

2. Katalysatorzusammensetzung gemäß Anspruch 1, worin b, c, d und f in der Formel (I) 0,7 bis 1,8, 0,5 bis 2,5, 5 bis 8 bzw. 0,02 bis 1, bezogen auf 12 Atome Molybdän, sind.

3. Katalysatorzusammensetzung gemäß Anspruch 1 oder 2, worin e in der Formel (I) 0,1 bis 2,5, bezogen auf 12 Atome Molybdän, ist.

4. Katalysatorzusammensetzung gemäß einem der Ansprüche 1 bis 3, worin Y in der Formel (I) Kalium ist.

5. Katalysatorzusammensetzung gemäß einem der Ansprüche 1 bis 3, worin Y in der Formel (I) Rubidium ist.

6. Katalysatorzusammensetzung gemäß einem der Ansprüche 1 bis 3, worin Y in der Formel (I) Cäsium ist.

7. Katalysatorzusammensetzung gemäß einem der Ansprüche 1 bis 3, worin Y in der Formel (I) ein Gemisch von mindestens 2 Elementen ist, die aus der aus Kalium, Rubidium und Cäsium bestehenden Gruppe ausgewählt sind.

8. Katalysatorzusammensetzung gemäß einem der Ansprüche 1 bis 7, die zusätzlich Siliciumdioxid als Träger enthält, der die Oxidkatalysatorzusammensetzung trägt, wobei der Siliciumdioxidträger in einer Menge von 30 bis 70 Gew.-%, bezogen auf das Gesamtgewicht der Oxidkatalysatorzusammensetzung und des Siliciumdioxidträgers, vorhanden ist.

9. Verfahren zur Herstellung von Acrylnitril aus Propylen oder von Methacrylnitril aus Isobuten oder tert-Butanol durch Ammoxidation des Propylens oder des Isobutens oder tert-Butanols, welches das Umsetzen von Propylen oder das Umsetzen von Isobuten oder tert-Butanol mit einem molekularen Sauerstoff enthaltenden Gas und Ammoniak bei einer Temperatur von 350°C bis 550°C unter einem Druck von Atmosphärendruck bis 3 atm in Anwesenheit einer Ammoxidationskatalysatorzusammensetzung gemäß einem der Ansprüche 1 bis 8 umfaßt.

10. Verfahren gemäß Anspruch 9, wobei das molekularen Sauerstoff enthaltende Gas Luft ist.

11. Verfahren gemäß Anspruch 9 oder 10, wobei die molaren Verhältnisse von Propylen oder Isobuten oder tert-Butanol: Ammoniak : Sauerstoff im Bereich von 1 : 0,8 bis 1,4 : 1,4 bis 2,4 sind.

12. Verfahren gemäß Anspruch 9, 10 oder 11, wobei die Umsetzung in einem Fluidbettreaktor durchgeführt wird.

## Revendications

1. Composition de catalyseur d'ammoxydation destinée à être utilisée pour préparer de l'acrylonitrile à partir du propylène ou du méthacrylonitrile à partir de l'isobutène ou du butanol tertiaire, par ammoxydation du propylène ou de l'isobutène ou du butanol tertiaire, comprenant une composition de catalyseur à base d'oxyde représentée par la formule (I) :

$$Mo_{12}(Bi_{1-a}Ce_a)_bFe_cNi_dX_eY_fO_g \qquad (I)$$

dans laquelle

X est au moins un élément choisi parmi le magnésium et le zinc,

Y est au moins un élément choisi parmi le potassium, le rubidium et le césium,

a est le rapport atomique du cérium à la somme du bismuth et du cérium,

b est le rapport atomique de la somme du bismuth et du cérium, relativement à douze atomes de molybdène, et c, d, e, f et g sont, respectivement, les rapports atomiques du fer, du nickel, du X, du Y et de l'oxygène relativement à douze atomes de molybdène,

a étant un nombre compris entre 0,6 et 0,8,
b étant un nombre compris entre 0,5 et 0,2,
c étant un nombre compris entre 0,1 et 3,
d étant un nombre compris entre 4 et 10,
e étant un nombre compris entre 0 et 3,

f étant un nombre compris entre 0,01 et 2, et
g étant un nombre déterminé par les exigences
de valence des autres éléments présents.

2. Composition de catalyseur suivant la revendication 1, dans laquelle b, c, d et f dans la formule (I) sont respectivement compris entre 0,7 et 1,8, entre 0,5 et 2,5, entre 5 et 8, et entre 0,02 et 1, relativement à douze atomes de molybdène.

3. Composition de catalyseur suivant la revendication 1 ou 2, dans laquelle e dans la formule (I) est compris entre 0,1 et 2,5, relativement à douze atomes de molybdène.

4. Composition de catalyseur suivant l'une quelconque des revendications 1 ou 3, dans laquelle Y dans la formule (I) est le potassium.

5. Composition de catalyseur suivant l'une quelconque des revendications 1 ou 3, dans laquelle Y dans la formule (I) est le rubidium.

6. Composition de catalyseur suivant l'une quelconque des revendications 1 ou 3, dans laquelle Y dans la formule (I) est le césium.

7. Composition de catalyseur suivant l'une quelconque des revendications 1 ou 3, dans laquelle Y dans la formule (I) est un mélange d'au moins deux éléments choisis dans le groupe consistant en le potassium, le rubidium et le césium.

8. Composition de catalyseur suivant l'une quelconque des revendications 1 ou 7, qui comprend en outre de la silice comme support qui supporte une composition de catalyseur à base d'oxyde, le support de silice représentant de 30 à 70 % du poids total de la composition de catalyseur à base d'oxyde et du support de silice.

9. Procédé de préparation d'acrylonitrile à partir du propylène ou de méthacrylonitrile à partir de l'isobutène ou du butanol tertiaire, par ammoxydation du propylène ou de l'isobutène ou du butanol tertiaire, qui consiste à faire réagir du propylène ou à faire réagir de l'isobutène ou du butanol tertiaire sur un gaz contenant de l'oxygène moléculaire et sur de l'ammoniac à une température de 350°C à 550°C sous une pression allant de la pression atmosphérique à trois atmosphères en la présence d'une composition de catalyseur d'ammoxydation telle que revendiquée suivant l'une quelconque des revendications 1 à 8.

10. Procédé suivant la revendication 9, dans lequel le gaz contenant de l'oxygène moléculaire est de l'air.

11. Procédé suivant la revendication 9 ou 10, dans lequel les rapports molaires du propylène ou de l'isobutène ou du butanol tertiaire : de l'ammoniac : de l'oxygène sont dans l'intervalle de 1:0,8 à 1,4:1,4 à 2,4.

12. Procédé suivant la revendication 9, 10 ou 11, qui consiste à effectuer la réaction dans un réacteur à lit fluidisé.